# EUROPEAN PATENT SPECIFICATION

(11) **EP 2 689 913 B1**
(45) Date of publication and mention of the grant of the patent: **14.03.2018**
(21) Application number: 13177885.4
(22) Date of filing: 24.07.2013
(51) Int. Cl.: B29C 49/42, A61L 2/12, A61L 2/18, B29C 49/46

(54) **System and method for the sterilisation of a preform**
System und Verfahren für die Sterilisation eines Vorformlings
Système et procédé pour la stérilisation d'une préforme

(30) Priority: 25.07.2012 IT PR20120049
(43) Date of publication of application: 29.01.2014
(73) Proprietor: Gea Procomac S.p.A., 43038 Sala Baganza (PR) (IT)
(72) Inventor: Pagliarini, Paolo, 43038 Sala Baganza (PR) (IT)
(74) Representative: Gotra, Stefano

(56) References cited:
- EP-A1- 2 295 324
- EP-B1- 0 721 808
- FR-A1- 2 766 121
- US-A1- 2010 047 120
- US-A1- 2011 285 063

## Description

The present invention relates to a method and an apparatus for the sterilisation of a preform.

An apparatus for the sterilisation of a preform of the type described in US2011/0272861 is known. This apparatus comprises a turntable placed upstream from a stretch blowing station of the preforms. The turntable comprises a plurality of gripping elements each intended to project within a corresponding preform in order to grasp it. The gripping elements each comprise a corresponding dispensing nozzle of a sterilising agent. Furthermore each preform can be moved by the gripping means to be introduced into an annular structure in which it is heated by microwaves. The sterilising agent is introduced when the heating of the preforms has already begun. This heating takes place by exposing the preforms to microwaves. Hence the sterilising agent comes into contact with the inside walls of the preform which are below the gripping means that obstruct the preform in proximity to the opening.

A drawback of this embodiment is connected with the constructive complexity of gripping means that integrate the dispensing nozzle of the sterilising agent within them and with the system needed to dose, vaporise, thermally insulate and distribute the sterilising agent on the rotating manifold. A further drawback of the embodiment indicated above is that the portions of the preform in proximity to the mouth are not subjected to a sterilisation process.

Document US2010/047120 discloses a method and a system for sterilising preforms according to preambles of claims 1 and 10, in which the neck of each preform is gripped by a bell that is lowered on the mouth of the preform. The bell comprises elastic claws for embracing the neck on the outside. Internally, the bell has a core that matches the inner surface of the neck. In this context, the technical task underpinning the present invention is to provide a method and an apparatus for the sterilisation of a preform, which obviate the drawbacks of the prior art cited above.

In particular, the object of the present invention is to provide a method and an apparatus for the sterilisation of a preform that allows simple dosing and improved distribution of the sterilising agent on the preform; this also translates into a reduction of the system complexity for the dosing system and the distribution of a sterilising agent (with a substantial reduction in the associated costs and a more extensive sterilising action and lower consumption of the sterilising agent). An important object of the present invention is that of sterilising not only the inside parts of the preform, but also parts of the preform located in proximity to the mouth. The technical task set and the objects specified are substantially attained by a method and an apparatus for the sterilisation of a preform, comprising the technical characteristics as set out in one or more of the accompanying claims.

Further characteristics and advantages of the present invention will more fully emerge from the non-limiting description of a preferred but not exclusive embodiment of an apparatus for the sterilisation of a preform, as illustrated in the accompanying drawings, in which:
- Figure 1 is a schematic plan view of an apparatus according to the present invention;
- Figures 2a-2g show a succession of steps of a method according to the present invention;
- Figures 3 and 4 show a perspective view and a sectional view respectively of a component of the apparatus according to the present invention;
- Figures 5 and 6 show a perspective view and a sectional view respectively of the component of Figures 3 and 4 in a distinct configuration.

The present invention relates to a system according to claim 10 comprising a preform 10 and an apparatus 1 for the sterilisation of a preform made of plastic material. In particular such apparatus 1 may be integrated into an apparatus for forming a container for food products starting from said preform. Typically the preform 10 comprises both a neck 13 equipped with an opening 11 and a bottom 12 opposite to the opening 11.

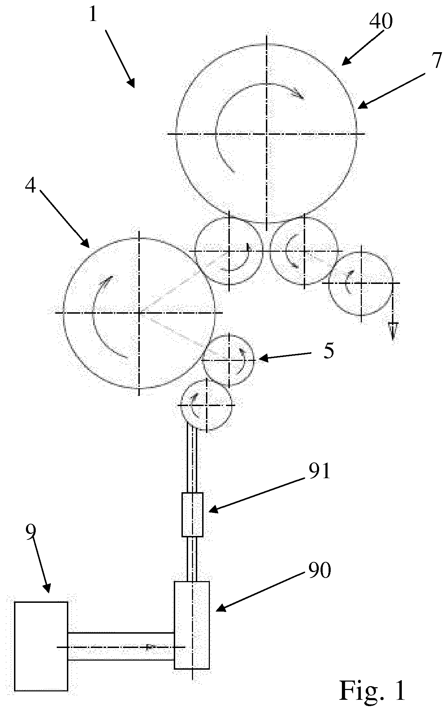

The apparatus 1 (see Figure 1) advantageously comprises a tank 9 from which the preforms are picked up. Downstream there are alignment means 90 of the preforms in one or more rows. Subsequently there are dust removal means 91 of the preforms. The tank 9, the alignment means 90 and the dust removal means 91 are of the known type and therefore not described further.

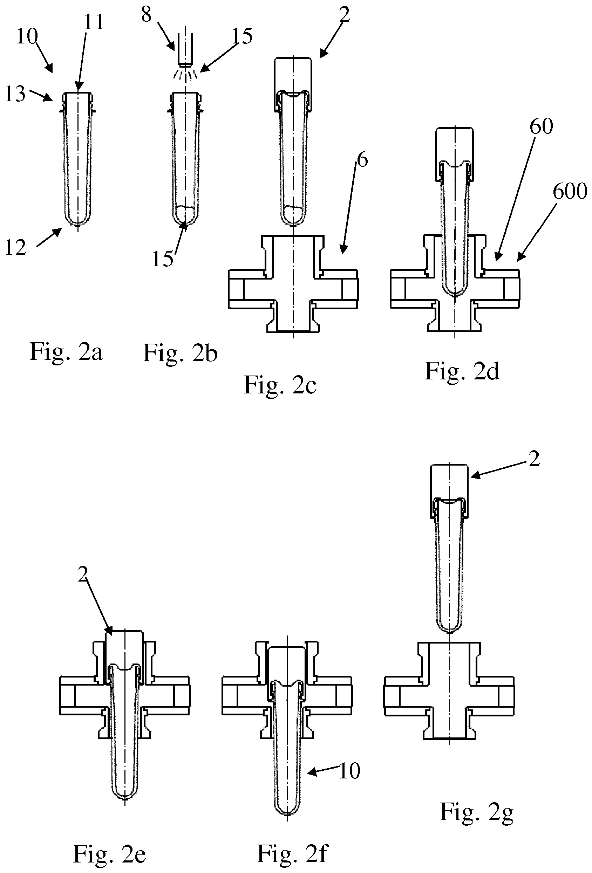

The apparatus 1 comprises introduction means 5 into the preform 10 of an agent 15 intended to perform a sterilising action (see Figure 2b). The agent 15 is introduced in the liquid state (even if during the introduction the sterilising agent 15 could be partially nebulised into tiny particles of liquid). Appropriately the introduction means 5 are placed downstream of the alignment means 90. These introduction means 5 advantageously comprise a dosing nozzle 8 of the known type. For example this dosing nozzle 8 may be of the type used for dosing/introducing liquid nitrogen into jars. The agent 15 is more sensitive to the heat generated by microwaves than the material of the preform 10. In particular the agent 15 is heat activatable. In particular at high temperatures the sterilising effect is increased. For example the agent 15 comprises hydrogen peroxide. In an alternative embodiment the agent 15 is water. In that case the sterilising action is connected with the fact that at high temperatures water (potentially in the form of vapour) also performs a sterilising action. Appropriately a movement line of the preforms transits at the nozzle 8, but the nozzle 8 remains in a predetermined position along the movement line. In other words, at least in the preferred embodiment the nozzle 8 does not move integrally with the preforms 10.

The apparatus 1 comprises heating means 60 of the agent 15 placed in or on the preform.

Appropriately the heating means 60 comprise microwave directing means 6 towards the preform 10. On this point the directing means 6 comprise a microwave applicator 600 (which by way of example is shaped like an annular element inside which the preform can transit). With reference to Figure 1, appropriately the directing means 6 rest on a rotating turntable 4, below also referred to as a rotating heating turntable 4. In the preferred embodiment the rotating turntable 4 comprises a plurality of heating stations each comprising corresponding microwave directing means 6. The heating stations are distributed along the perimeter of the turntable 4. Each heating station is associated with corresponding gripping means 2. With respect to a movement path of the preform along the apparatus 1, the introduction means 5 into the preform of the agent 15 are placed upstream from the microwave directing means 6 towards the preform. The apparatus 1 (preferably the rotating turntable 4) also comprises gripping means 2 of the preform 10. The gripping means 2 identify a cavity 3 in combination with the preform 10 itself. Appropriately the cavity 3 is annular and externally surrounds the neck 13 of the preform 10. The cavity 3 is in fluid communication with the inside of the preform 10. The gripping means 2 comprise a gripper 20 better described below.

Appropriately it is the movement of the gripping means 2 that allows the preforms to move towards and away from the corresponding microwave directing means 6. Although in the preferred embodiment the heating means 60 comprise said microwave applicator 600, they could alternatively also use other sources of energy (for example emitters of other types of electromagnetic waves).

The apparatus 1 further comprises a forming station 7 (typically a blowing or stretch-blowing one). The forming station 7 is placed downstream from the microwave directing means 6. Preferably the station 7 rests on a dedicated turntable 40. The present invention further comprises a method for the sterilisation of a preform 10 made of plastic material according to claim 1. Appropriately this method is implemented by an apparatus 1 having one or more of the technical characteristics described above.

The preform 10 comprises a neck 13 equipped with an opening 11 and a bottom 12 opposite to the opening 11.

Appropriately the method envisages the following preliminary steps (please refer to Figure 1):
- taking a succession of preforms 10 from a tank 9;
- putting the preforms 10 in order according to a movement row;
- performing a dust removal procedure.

The method comprises the steps of introducing into the preform 10 an agent 15 intended to perform a sterilising action on the preform (see Figure 2b). This advantageously takes place after performing the preliminary steps indicated above. The step of introducing the sterilising agent 15 envisages dispensing the sterilising agent 15 through a nozzle 8 (advantageously of the type described above). During said step of introducing the sterilising agent 15 the nozzle 8 remains still while the preforms 10 move below it.

The sterilising agent 15 is introduced into the preform 10 in liquid form.

The method therefore envisages the step of heating the sterilising agent 15 contained in the preform 10 in order to cause its evaporation (compare Figures 2c and 2d).

Appropriately the step of heating the sterilising agent 15 envisages directing microwaves towards the preform 10. The description with reference to the microwaves can be repeated for other types of electromagnetic radiation. In this way the quick movement of the dipoles of the sterilising agent 15 induced by the microwaves causes quicker heating of the sterilising agent 15 with respect to the preform (usually made of PET).

The step of directing the microwaves towards the preform 10 takes place advantageously in the heating turntable 4.

The sterilising agent 15, if treated with microwaves, heats up more quickly than the material of the preform 10 (the preforms are usually made of PET). The sterilising agent 15 advantageously comprises/is hydrogen peroxide. The latter is approximately 100 times more sensitive to microwaves than PET.

The step of introducing said sterilising agent 15 takes place before directing the microwaves towards the preform 10 (see Figure 1) and envisages collecting said sterilising agent 15 on the bottom 12 of the preform 10 (see Figure 2b). The sterilising agent 15 just introduced accumulates due to gravity on the bottom 12 of the preform 10 (see Fig. 2c).

Just before the start of the step of directing the microwaves towards the preform 10, the sterilising agent 15 is completely or almost completely on the bottom 12 of the preform 10.

The step of introducing the sterilising agent 15 into the preform 10 takes place upstream from the heating turntable 4.

The step of directing the microwaves towards the preform 10 envisages the preform being introduced into the microwave applicator 600. The step of directing the microwaves towards the preform 10 therefore envisages (causes) the evaporation of the sterilising agent 15. The step of directing the microwaves towards the preform 10 first envisages the exposure to the microwaves only of the bottom of the preform 10 and of the sterilising agent 15 collected on the bottom 12. Only subsequently will the exposure to the microwaves of the remaining parts of the preform take place. The evaporation of the sterilising agent 15 by the microwaves is instantaneous (it usually takes less than 1 second, even if this depends on the powers at stake and the mass of the agent 15). Because of the reasons indicated above, when the sterilising agent 15 placed on the bottom 12 evaporates the preform 10 will be colder than the sterilising agent 15. Consequently, the sterilising agent 15 condenses in contact with the preform 10. The condensation of the sterilising agent 15 on the perform affects the entire inside surface 101 of the preform 10 and part of the outside surface 102 of the preform 10. The sterilising agent 15 that condenses on the preform is more concentrated (in particular the percentage of the sterilising component, for example, hydrogen peroxide, is higher than on the bottom 12 of the preform before evaporation).

As mentioned above, the step of introducing the preform into the applicator 600 takes place by inserting the bottom 12 of the preform first (see Figures 2c and 2d). In this way the sterilising agent 15 located there evaporates before the substantial heating of other parts of the preform 10 (so the evaporated sterilising agent finds the preform still at a low temperature which facilitates the subsequent condensation). The evaporation step of the sterilising agent 15 collected in the preform 10 therefore causes part of the sterilising agent 15 to exit through the opening 11 in the preform 10 and the condensation of the sterilising agent 15 onto an outside portion of the preform 10. The evaporation step upwards of the sterilising agent from the bottom 12 of the preform 10 is important since it promotes the optimal distribution thereof along all the inside walls of the preform and thanks to the annular cavity 3 also at the outside surface of the neck 13.

The method further envisages the re-evaporation of the sterilising agent 15 condensed on the preform 10 (following the previous evaporation). This always happens by directing the microwaves towards the preform. In the event that the sterilising agent 15 comprises hydrogen peroxide this can be accompanied by the activation of the agent 15 and appropriately its distribution. Appropriately the step of directing microwaves towards the preform 10 envisages the softening of the preform 10 itself for its shaping (there is a significant softening of the preform only following the re-evaporation of the sterilising agent 15). In this way the energy of the microwaves is used both for the re-evaporation of the sterilising agent 15, and for the heating of the preform 10 according to a predetermined temperature profile. On this point the sterilisation method can be integrated into a method for forming a container starting from a preform. After the condensation of the sterilising agent 15 on the outside of the neck 13 the latter is completely introduced into the annular applicator 600 to allow the re-evaporation of the sterilising agent 15 condensed there (see Figure 2f).

The step of heating the agent 15 contained in the preform 10 (i.e. preferably directing the microwaves towards the preform 10) is preceded by a step of gripping the preform 10 by gripping means 2 acting on the outside of the preform 10 (see Figure 2b). Appropriately the gripping means 2 identify, in combination with the preform 10 itself, an annular cavity 3. The annular cavity 3 externally surrounds the neck 13 of the preform and is in fluid communication with the inside of the preform 10 to allow the evaporated sterilising agent 15 to reach the outside of the neck 13.

Appropriately at least a part of the evaporated sterilising agent 15 recondenses on the outside of the neck 13 of the preform 10.

Usually the outside of the neck 13 comprises a threaded part 17 (for example intended for connection with an external cap) and preferably the evaporated sterilising agent 15 is distributed across the whole of said threaded part 17 by re-condensing.

The gripping means 2 (which for example comprise a gripper 20) engage below a first protrusion 18 of the preform 10 which develops along an annular line surrounding the preform 10. In the technical field the first protrusion 18 is also known as a seal tearing ring. Preferably the whole weight of the preform 10 is unloaded onto said gripper 20.

Appropriately the gripper 20 engages in a groove 93 interposed between said first protrusion 18 and a second protrusion 94. This second protrusion 94 also develops along an annular line that surrounds the preform 10. In the technical field the second protrusion 94 is also known as a neck rim. The gripping step ensures that the preform hangs from the gripping means 2.

In particular the gripping step envisages the gripper 20 entirely surrounding the threaded part 17 of the preform 10.

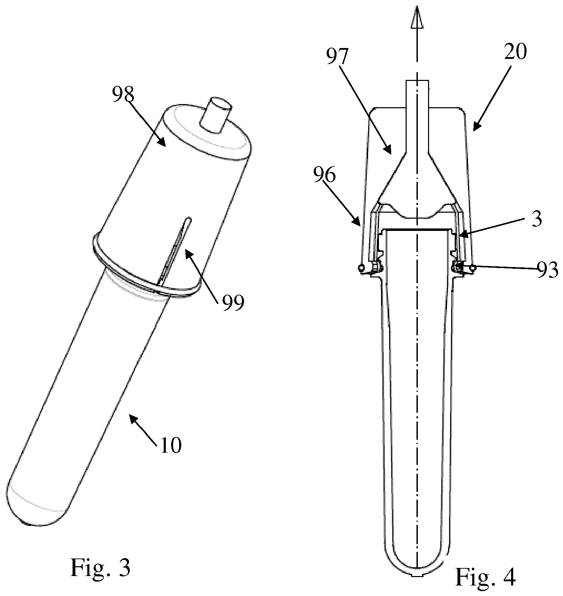

In the preferred embodiment the gripper 20 is made of PTFE. The opening and closing of the gripper 20 may be of various kinds. Advantageously the gripper 20 comprises a shell 95 which is open at one end (typically the lower end) for the insertion of the preform 10. Appropriately the shell is equipped with a plurality of jaws 96 intended to surround at least a part of the preform 10. The jaws 96 project in a cantilever fashion from a support 98 being part of the shell 95. The shell 95 has one or more weakening grooves 99 that advantageously separate the jaws 96 from each other. The gripping step envisages a divarication of the gripping means 2 (typically a divarication of the shell 95), see, for example, Figure 4.

The gripping step further comprises the introduction of the neck 13 of the preform inside a shell 95 defined by the gripping means 2.

In particular the divarication step envisages an elastic deformation of the jaws 96 of the gripper 20.

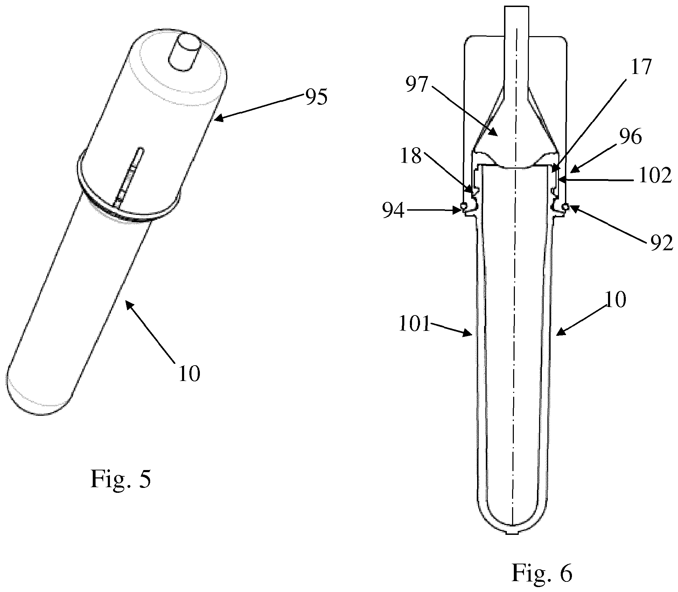

The gripping step further comprises a tightening step on the preform 10 of the gripping means 2. This tightening action comprises an elastic return of the gripping means 2 (see Figure 6). Advantageously the gripper 20 comprises an elastic ring 92 which is in contact with and surrounds the shell 95 (in particular the jaws 96). The divarication step of the shell 95 envisages a deformation of the ring 92 which during the tightening step returns potential elastic energy stored in the divarication step (this detail allows greater tightening force towards the preform 10).

During the tightening step the jaws 96 are inserted into said groove 93. To allow the divarication of the jaws the gripper 20 may comprise a divaricator 97 sliding along a first direction; this sliding divaricator 97 is mobile between a first position in which it is inside the shell 95 and determines the reciprocal movement of the jaws 96 away from each other and a second position in which it is inside the shell and is disengaged from the jaws enabling them to move towards each other.

With respect to the shell 95 the divaricator 97 in the first position is higher than in the second position. The divaricator 97 can therefore move integrally with the shell 95 in order to allow a shift of the gripper 20, but can also move in relation to the shell 95 to allow the divarication and tightening of the jaws 96. Appropriately the divaricator 97 comprises a conical surface which can be operatively coupled with a conical surface of the shell 95. The conical surface of the shell 95 has a higher angle of convergence than the angle of convergence of the conical surface of the shell 95. In order to perform the divarication step of the shell 95 the divaricator 97 is raised with respect to the shell 95 and increases the contact area between the conical surface of the divaricator 97 and the conical surface of the shell 95.

In particular the gripping step envisages the gripper 20 surrounding the threaded part 17 of the preform 10. In a particular embodiment (see Figure 2) the gripping step envisages a part of the gripper 20 projecting into the preform 10 (without coming into contact with the preform 10 and however without completely obstructing the opening 11). Appropriately the part of the gripper 20 that projects into the preform 10 is tapered in order to facilitate the passage of the vaporised sterilising agent 15 from the inside of the preform 10 to the annular cavity 3.

After the softening of the preform 10, which takes place in the heating turntable 4, the preform is subjected to a deformation step for the forming of the final container. The forming step mainly takes place by blowing and/or stretch blowing. Typically the forming step takes place in a forming turntable 40 separate from the heating turntable 4.

The invention enables multiple advantages to be attained.

First of all, it allows optimal distribution of the sterilising agent thanks to the evaporation action from the lower bottom of the preform. This optimal distribution is also enabled due to the fact that the preform is externally gripped and therefore there are no obstacles to the distribution of the sterilising agent within the preform, but neither are there any obstacles that prevent the exit of the sterilising agent in the annular cavity 3.

The disclosure as conceived is susceptible to numerous modifications and variants. Furthermore all the details can be replaced by other technically equivalent element. In practice, all the materials used, as well as the dimensions, can be any according to requirements.

## Claims

1. Method for the sterilisation of a preform (10) made of plastic material, said preform comprising both a neck (13) equipped with an opening (11) and a bottom (12) opposite to the opening (11), the method comprising the steps of:
- introducing into the preform (10) a sterilising agent (15) for a sterilising action;
- heating the agent (15) contained in the preform (10) to determine its evaporation, the step of heating the agent (15) contained in the preform (10) being preceded by a gripping step of the preform (10) using gripping means (2) acting on the outside of the preform (10); **characterized in that** said gripping means (2) identifying, in combination with the preform (10) itself, an annular cavity (3), said annular cavity (3) externally surrounding the neck (13) of the preform (10), said annular cavity (3) is in fluid communication with the inside of the preform (10) so as to allow the evaporated agent (15) to reach the inside and the outside of the neck (13).

2. Method according to claim 1, **characterised in that** the gripping step envisages:
- divarication of the gripping means (2);
- the introduction into the neck (13) of the preform inside a shell (95) defined by the gripping means (2);
- a tightening step on the preform (10) of the gripping means (2), such gripping action comprising an elastic return of the gripping means (2).

3. Method according to claim 2, **characterised in that** the divarication step is determined by a divaricator (97) inside the shell which by moving from a second to a first position induces the reciprocal movement away from each other of jaws (96) being part of the shell (95); during the tightening step the divaricator (97) moves from the first to the second position to disengage from the jaws (96) allowing reciprocal movement towards each other.

4. Method according to any one of the previous claims, **characterised in that** the evaporated agent (15) partially recondenses on the outside of the neck (13) of the preform (10).

5. Method according to claim 4, **characterised in that** by recondensing the evaporated agent (15), the latter is distributed wholly across a threaded part (17) of the outside of the neck (13) that is connectable with an external cap.

6. Method according to any one of the previous claims, **characterised in that** the gripping step envisages that the gripping means (2) are engaged below a projection (18) of the preform which extends along the annular line surrounding the preform (10), the entire weight of the preform (10) being unloaded onto said gripping means (2).

7. Method according to any one of the previous claims, **characterised in that** the step of introducing the agent (15) in the preform (10) envisages that the latter accumulates by gravity in liquid form on the bottom (12) of the preform (10); the step of heating the agent (15) contained in the preform to determine its evaporation envisages directing microwaves towards the preform (10).

8. Method according to claim 7, **characterised in that** the step of directing the microwaves towards the preform (10) envisages the step of introducing the preform in the microwave applicator (600); the step of introducing the preform into the applicator (600) takes place by inserting first the bottom (12) into the applicator (600).

9. Method according to claim 8 when it depends directly or indirectly on claim 2, **characterised in that** at least after the condensation of the agent (15) on the outside of the neck the gripping means (2) and the neck (13) of the preform (10) are introduced into the microwave applicator (600) to re-evaporate the agent (15).

10. System comprising:
i) a preform (10) made of plastic material, said preform (10) comprising both a neck (13) equipped with an opening (11) and a bottom (12) opposite to the opening (11);
ii) an apparatus (1) for the sterilisation of a preform (10), said apparatus (1) comprising:
- introduction means (5) into the preform (10) of sterilising agent (15) for a sterilising action;
- heating means (60) of the agent contained in the preform;
**characterized in that** it comprises
- gripping means (2) of the preform (10) which identify, in combination with the preform itself, an annular cavity (3),
said annular cavity (3) externally surrounding the neck (13) of the preform (10), said annular cavity (3) is in fluid communication with the inside of the preform (10).

## Patentansprüche

1. Verfahren für die Sterilisation eines Vorformlings (10) aus Kunststoffmaterial, wobei der Vorformling sowohl einen mit einer Öffnung (11) versehenen Hals (13) als auch einen der Öffnung (11) gegenüberliegenden Boden (12) umfasst, wobei das Verfahren die Schritte umfasst von:
- Einführen eines Sterilisierungsmittels (15) in den Vorformling (10) für eine sterilisierende Wirkung;
- Erhitzen des im Vorformling (10) enthaltenen Mittels (15), um dessen Verdampfung zu bestimmen, wobei dem Schritt des Erhitzens des im Vorformling (10) enthaltenen Mittels (15) ein Greifschritt des Vorformlings (10) unter Verwendung von auf die Aussenseite des Vorformlings (10) einwirkenden Greifmitteln (2) vorausgeht; **dadurch gekennzeichnet, dass** die Greifmittel (2) in Kombination mit dem Vorformling (10) selbst einen ringförmigen Hohlraum (3) identifizieren, wobei der ringförmige Hohlraum (3) den Hals (13) des Vorformlings (10) aussen umgibt,
der ringförmige Hohlraum (3) steht in Fluidverbindung mit dem Inneren des Vorformlings (10), so dass das verdampfte Mittel (15) das Innere und die Aussenseite des Halses (13) erreichen kann.

2. Verfahren nach Anspruch 1, **dadurch gekennzeichnet, dass** der Greifschritt vorsieht:
- Spreizen der Greifmittel (2);
- Einführen des Vorformlings in den Hals (13) in eine Schale (95), die durch die Greifmittel (2) definiert wird;
- einen Festziehschritt der Greifmittel (2) am Vorformling (10), wobei eine solche Greifwirkung eine elastische Rückkehr der Greifmittel (2) umfasst.

3. Verfahren nach Anspruch 2, **dadurch gekennzeichnet, dass** der Spreizschritt durch eine Spreizvorrichtung (97) innerhalb der Schale bestimmt wird, die durch die Bewegung von einer zweiten in eine erste Position die gegenseitige voneinander wegführende Bewegung der Backen (96), die Teil der Schale (95) sind, veranlasst; während des Festziehschritts bewegt sich die Spreizvorrichtung (97) von der ersten in die zweite Position, um sich von den Backen (96) zu lösen und um eine gegenseitige Bewegung zueinander zu ermöglichen.

4. Verfahren nach einem der vorhergehenden Ansprüche, **dadurch gekennzeichnet, dass** das verdampfte Mittel (15) teilweise auf der Aussenseite des Halses (13) des Vorformlings (10) wieder kondensiert.

5. Verfahren nach Anspruch 4, **dadurch gekennzeichnet, dass** das verdampfte Mittel (15) durch Rekondensation vollständig über einen Gewindeteil (17) der Aussenseite des Halses (13) verteilt wird, der mit einer äusseren Kappe verbindbar ist.

6. Verfahren nach einem der vorhergehenden Ansprüche, **dadurch gekennzeichnet, dass** der Greifschritt vorsieht, dass die Greifmittel (2) unterhalb eines Vorsprungs (18) des Vorformlings, der sich entlang der den Vorformling (10) umgebenden ringförmigen Linie erstreckt, eingreifen, wobei das ganze Gewicht des Vorformlings (10) auf die Greifmittel (2) entladen wird.

7. Verfahren nach einem der vorhergehenden Ansprüche, **dadurch gekennzeichnet, dass** der Schritt des Einführens des Mittels (15) in den Vorformling (10) vorsieht, dass sich letzterer durch Schwerkraft in flüssiger Form auf dem Boden (12) des Vorformlings (10) ansammelt; der Schritt des Erhitzens des im Vorformling enthaltenen Mittels (15) zur Bestimmung dessen Verdampfung sieht vor, dass Mikrowellen auf den Vorformling (10) gerichtet werden.

8. Verfahren nach Anspruch 7, **dadurch gekennzeichnet, dass** der Schritt des Richtens der Mikrowellen in Richtung des Vorformlings (10) den Schritt des Einführens des Vorformlings in den Mikrowellenapplikator (600) vorsieht; der Schritt des Einführens des Vorformlings in den Applikator (600) erfolgt zuerst durch Einsetzen des Bodens (12) in den Applikator (600).

9. Verfahren nach Anspruch 8, wenn er direkt oder indirekt vom Anspruch 2 abhängt, **dadurch gekennzeichnet, dass** zumindest nach der Kondensation des Mittels (15) auf der Aussenseite des Halses das Greifmittel (2) und der Hals (13) des Vorformlings (10) in den Mikrowellenapplikator (600) eingeführt werden, um das Mittel (15) wieder zu verdampfen.

10. System, umfassend:
i) einen Vorformling (10) aus Kunststoffmaterial, wobei der Vorformling (10) sowohl einen mit einer Öffnung (11) versehenen Hals (13) als auch einen der Öffnung (11) gegenüberliegenden Boden (12) umfasst;
ii) eine Vorrichtung (1) für die Sterilisation eines Vorformlings (10), wobei die Vorrichtung (1) umfasst:
- Einführungsmittel (5) in den Vorformling (10) des Sterilisationsmittels (15) für eine sterilisierende Wirkung;
- Heizmittel (60) des im Vorformling enthaltenen Mittels; **dadurch gekennzeichnet, dass** es umfasst
- Greifmittel (2) des Vorformlings (10), die in Kombination mit dem Vorformling selbst einen ringförmigen Hohlraum (3) identifizieren, wobei der ringförmige Hohlraum (3) den Hals (13) des Vorformlings (10) aussen umgibt, der ringförmige Hohlraum (3) steht in Fluidverbindung mit dem Inneren des Vorformlings (10).

## Revendications

1. Procédé pour la stérilisation d'une préforme (10) en matière plastique, ladite préforme comprenant un col (13), doté d'une ouverture (11), et un fond (12) opposé à l'ouverture (11), le procédé comprenant les étapes de :
- introduire dans la préforme (10) un agent stérilisant (15) pour effectuer une action stérilisante ;
- chauffer l'agent (15) contenu dans la préforme (10) pour déterminer son évaporation, l'étape de chauffage de l'agent (15) contenu dans la préforme (10) étant précédée par une étape de préhension de la préforme (10) en utilisant des moyens de préhension (2) agissant sur l'extérieur de la préforme (10) ; **caractérisé en ce que** lesdits moyens de préhension (2) identifient, en combinaison avec la préforme (10) elle-même, une cavité annulaire (3), ladite cavité annulaire (3) entourant extérieurement le col (13) de la préforme (10),
ladite cavité annulaire (3) est en communication fluidique avec l'intérieur de la préforme (10) de sorte à permettre à l'agent évaporé (15) d'atteindre l'intérieur et l'extérieur du col (13).

2. Procédé selon la revendication 1, **caractérisé en ce que** l'étape de préhension prévoit :
- l'écartement des moyens de préhension (2) ;
- l'introduction du col (13) de la préforme à l'intérieur d'une coque (95) définie par les moyens de préhension (2) ;
- une étape de serrage sur la préforme (10) des moyens de préhension (2), ladite action de préhension comprenant un retour élastique des moyens de préhension (2).

3. Procédé selon la revendication 2, **caractérisé en ce que** l'étape d'écartement est déterminée par un écarteur (97) à l'intérieur de la coque qui, par le déplacement d'une seconde à une première position, induit l'éloignement réciproque des mâchoires (96) faisant partie de la coque (95) ; lors de l'étape de serrage, l'écarteur (97) se déplace de la première à la seconde position pour se désengager des mâchoires (96) permettant leur rapprochement réciproque.

4. Procédé selon l'une quelconque des revendications précédentes, **caractérisé en ce que** l'agent évaporé (15) se condense de nouveau partiellement sur l'extérieur du col (13) de la préforme (10).

5. Procédé selon la revendication 4, **caractérisé en ce qu'**en condensant de nouveau l'agent évaporé (15), ce dernier est réparti entièrement à travers une partie filetée (17) de l'extérieur du col (13) pouvant être raccordé à un capuchon externe.

6. Procédé selon l'une quelconque des revendications précédentes, **caractérisé en ce que** l'étape de préhension prévoit que les moyens de préhension (2) se mettent en prise en dessous d'une saillie (18) de la préforme se prolongeant le long de la ligne annulaire entourant la préforme (10), la totalité du poids de la préforme (10) étant déchargé sur lesdits moyens de préhension (2).

7. Procédé selon l'une quelconque des revendications précédentes, **caractérisé en ce que** l'étape consistant à introduire l'agent (15) dans la préforme (10) prévoit que ce dernier s'accumule par gravité sous forme liquide sur le fond (12) de la préforme (10) ; l'étape de chauffage de l'agent (15) contenu dans la préforme pour déterminer son évaporation prévoit de diriger des micro-ondes vers la préforme (10).

8. Procédé selon la revendication 7, **caractérisé en ce que** l'étape consistant à diriger les micro-ondes vers la préforme (10) prévoit l'étape d'introduction de la préforme dans l'applicateur de micro-ondes (600) ; l'étape d'introduction de la préforme dans l'applicateur (600) se déroule en introduisant d'abord le fond (12) dans l'applicateur (600).

9. Procédé selon la revendication 8 lorsqu'elle dépend directement ou indirectement de la revendication 2, **caractérisé en ce qu'**au moins après la condensation de l'agent (15) sur l'extérieur du col, les moyens de préhension (2) et le col (13) de la préforme (10) sont introduits dans l'applicateur de micro-ondes (600) pour faire évaporer de nouveau l'agent (15).

10. Système comprenant :
i) une préforme (10) en matière plastique, ladite préforme (10) comprenant un col (13), doté d'une ouverture (11), et un fond (12) opposé à l'ouverture (11) ;
ii) un appareil (1) pour la stérilisation d'une préforme (10), ledit appareil (1) comprenant :
- des moyens d'introduction (5) dans la préforme (10) d'un agent stérilisant (15) pour effectuer une action stérilisante ;
- des moyens de chauffage (60) de l'agent contenu dans la préforme ; **caractérisé en ce qu'**il comprend
- des moyens de préhension (2) de la préforme (10) qui identifient, en combinaison avec la préforme elle-même, une cavité annulaire (3), ladite cavité annulaire (3) entourant extérieurement le col (13) de la préforme (10), ladite cavité annulaire (3) est en communication fluidique avec l'intérieur de la préforme (10).
